# EUROPEAN PATENT SPECIFICATION

(11) **EP 1 844 010 B1**
(45) Date of publication and mention of the grant of the patent: **13.02.2013**
(21) Application number: 06722936.9
(22) Date of filing: 08.05.2006
(51) Int. Cl.: C07F 7/18, C07C 401/00, C07B 37/08

(54) **ISOMERISATION OF PHARMACEUTICAL INTERMEDIATES**
ISOMERISIERUNG PHARMAZEUTISCHER ZWISCHENPRODUKTE
ISOMÉRISATION D'INTERMÉDIAIRES PHARMACEUTIQUES

(30) Priority: 17.03.2006 US 783076 P
(43) Date of publication of application: 17.10.2007
(73) Proprietor: LEO PHARMA A/S, 2750 Ballerup (DK)
(72) Inventor: FOLKMANN, Michael, Peter, DK-2840 Holte (DK); HANSEN, Erik, Torngaard, DK-3390 Hundested (DK)
(86) International application number: PCT/DK2006/000244
(87) International publication number: WO 2007/082533

(56) References cited:
- WO-A-2004/037781
- WO-A2-2005/095336
- D.M.ASHCROFT ET AL: "Systematic Review of Comparative Efficacy and Tolerability of Calcipotriol in Treating Chronic Plaque Psoriasis" BRIT.MED.J., vol. 320, 2000, pages 963-967, XP009077880
- CALVERLEY M J: "SYNTHESIS OF MC 903, A BIOLOGICALLY ACTIVE VITAMIN D METABOLITE ANALOGUE" TETRAHEDRON, ELSEVIER SCIENCE PUBLISHERS, AMSTERDAM, NL, vol. 43, no. 20, 1987, pages 4609-4619, XP001147723 ISSN: 0040-4020 cited in the application
- STEINMEYER A ET AL: "Synthesis and biological activities of a new series of secosteroids: Vitamin D phosphonate hybrids" STEROIDS, BUTTERWORTH-HEINEMANN, STONEHAM, MA, US, vol. 66, no. 3-5, March 2001 (2001-03), pages 257-266, XP002328551 ISSN: 0039-128X

## Description

### FIELD OF THE INVENTION

The present invention relates to an isomerisation method of vitamin D analogues useful for the synthesis of calcipotriol {(5Z, 7E, 22E, 24S)-24-cyclopropyl-9,10-secochola-5,7,10(19),22-tetraene-1α-3β-24-triol}, and to the use of a flow-through or continuous flow photoreactor for making said vitamin D analogues. The present invention relates further to the use of intermediates produced with said method for making calcipotriol or calcipotriol monohydrate, or pharmaceutical formulations thereof.

### BACKGROUND OF THE INVENTION

Calcipotriol or calcipotriene (structure I) [CAS 112965-21-6] shows a strong activity in inhibiting undesirable proliferation of epidermal keratinocytes [F.A.C.M. Castelijins, M.J. Gerritsen, I.M.J.J. van Vlijmen-Willems, P.J. van Erp, P.C.M. van de Kerkhof; Acta Derm. Venereol. 79, 11, 1999]. The efficiency of calcipotriol and calcipotriol monohydrate (I-hydrate) in the treatment of psoriasis was shown in a number of clinical trials [D.M. Ashcroft et al.; Brit. Med. J. 320, 963-67, 2000] and calcipotriol is currently used in several commercial drug formulations.

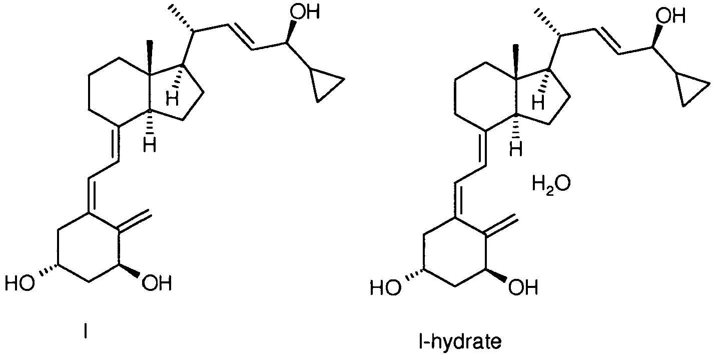

In the preparation of calcipotriol, the (Z)-stereochemistry for the double bond at C-5 is necessary for full expression of the biological activity. In the previously disclosed process for making calcipotriol I, the hydroxyl protected intermediate IIaaa with (*E*)-stereochemistry at C-5 is photoisomerised in an unspecified process on a laboratory scale using anthracene as a photocatalyst to give the corresponding (Z)-isomer IIIaaa followed by the removal of the silyl protecting groups to give calcipotriol I [WO 87/00834, M.J. Calverley; Tetrahedron, 43 (20), 4609-19, 1987; E. Binderup, Drugs of the Future Vol. 15, No. 1, 1990, "Calcipotriol", M.P. Folkmann, Ph.D. Thesis, The Danish Academy of Technical Science (ATV) EF 488, 1996].

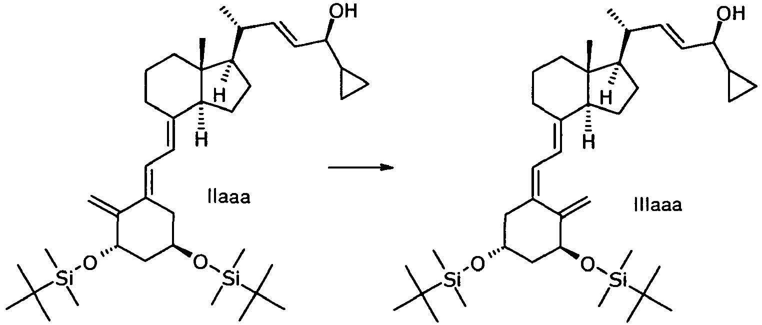

The references above do not teach how to scale the isomerisation of IIaaa or related compounds to achieve a process applicable to large-scale production. Hence, a routine process applicable to large-scale production for the isomerisation of vitamin D analogues useful is the synthesis of calcipotriol is needed.

The problems associated with performing preparative synthetic photochemistry on large scale have been perceived as being preventive to its routine application on an industrial scale. Photochemical conversions are in general difficult to scale, if at all. The utility of a specific photochemical reaction also often depends on specific reactor and light source design, among many other variables, which are all scale-dependent.

### SUMMARY OF THE INVENTION

The present invention relates to a process, suitable for large scale production, for the photoisomerisation vitamin D-analogues which are useful in the synthesis of calcipotriol. The present inventors have surprisingly found that by using a flow photoreactor, e.g. a flow-through photoreactor or continuous flow photoreactor, the desired 5-(Z)-isomers of general structures IIIa, IIIb, IIIc, and IIId respectively, can be obtained in a convenient large-scale production process in good yield. In direct comparison to a method which uses a fixed volume batch reactor the method of the present invention may furthermore result in reduced irradiation time and to photoisomerisation products with improved purity.

In one aspect, this invention relates to a method of isomerising a solution of a vitamin D derivative of general structure IIa, IIb, IIc, IId or IIe respectively; 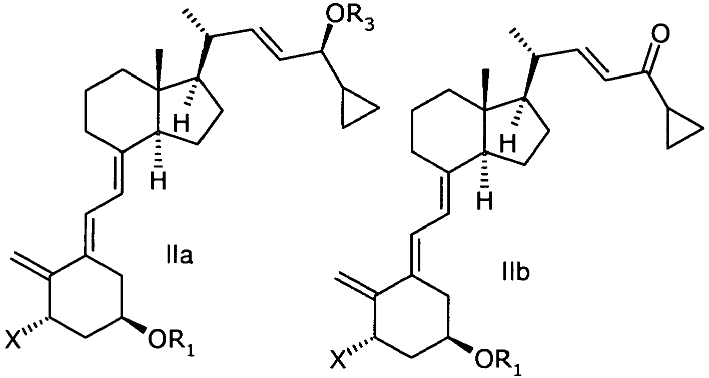 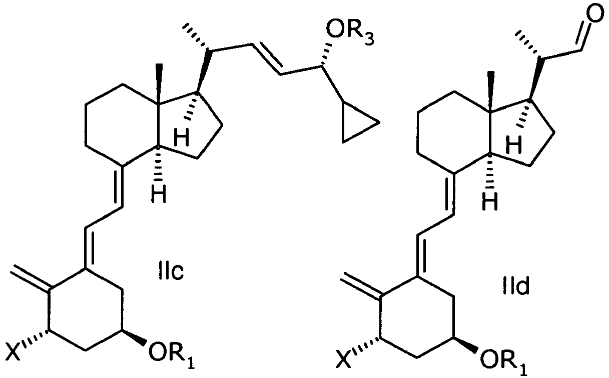 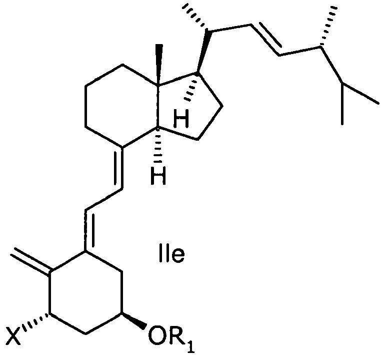 to give a vitamin D derivative of general structure IIIa, IIIb, IIIc, IIId, or IIIe respectively; 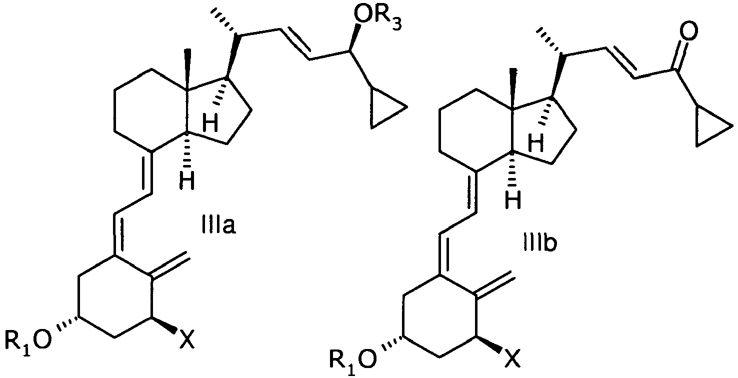 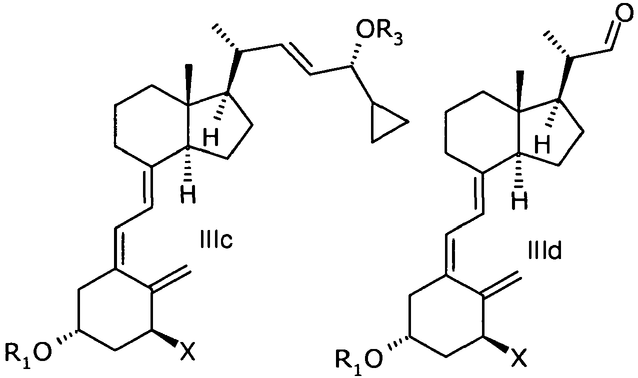 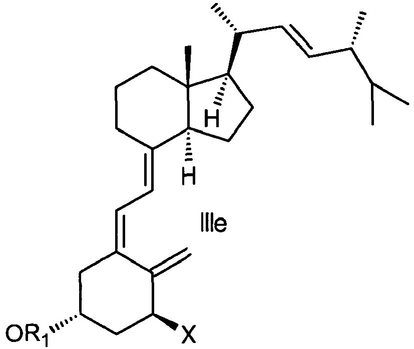 wherein X represents hydrogen or -OR₂;
R₁, R₂ and R₃ may be the same or different and independently represent hydrogen or a hydroxy protecting group;
the method comprising the irradiation of a solution of a vitamin D derivative of general structure IIa, IIb, IIc, IId or IIe respectively,
with a suitable light source in the presence of a triplet sensitizer with a triplet energy in the range of 150-270 kJ/mol in a flow-through photoreactor or continuous flow photoreactor, wherein said solution is moving in a single pass or circulating in multiple pass continuous flow relatively to the light source in a flow-through photoreactor or continuous flow photoreactor.

In another aspect, this invention relates to a method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of
(i) isomerising a vitamin D derivative of general structure IIaa 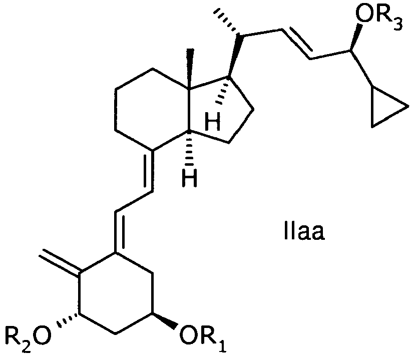 to give a vitamin D derivative of general structure IIIaa; 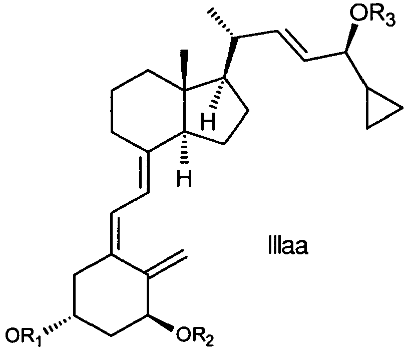 wherein R₁, R₂ and R₃ may be the same or different and independently represent hydrogen or a hydroxy protecting group;
   with a suitable light source in the presence of a triplet sensitizer with a triplet energy in the range of 150-270 kJ/mol; characterised in that said solution is moving in single pass or circulating in multiple pass continuous flow relatively to the light source in the flow-through photoreactor or continuous flow photoreactor;
(ii) when R₁ and/or R₂ and/or R₃ are not hydrogen, removing the hydroxy protecting group(s) R₁ and/or R₂ and/or R₃ of the compound of general structure IIIa to generate calcipotriol ; and
(iv) optionally crystallising the calcipotriol from a mixture of an organic solvent and water to give calcipotriol monohydrate.

In yet another aspect, this invention relates to a method of preparing calcipotriol or calcipotriol monohydrate comprising in one or more steps the method above.

In yet another aspect, this invention relates to a method of isomerising a solution of a vitamin D derivative of general structure IIaaa; 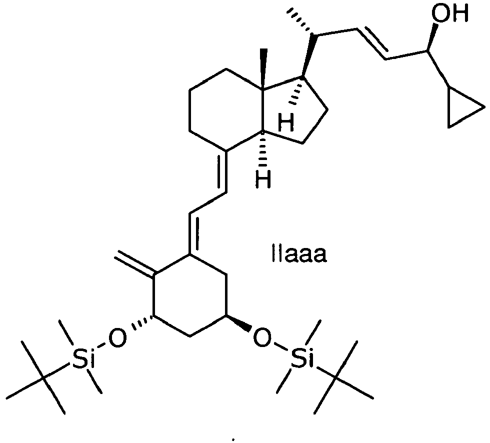 to give a vitamin D derivative of general structure IIIaaa, 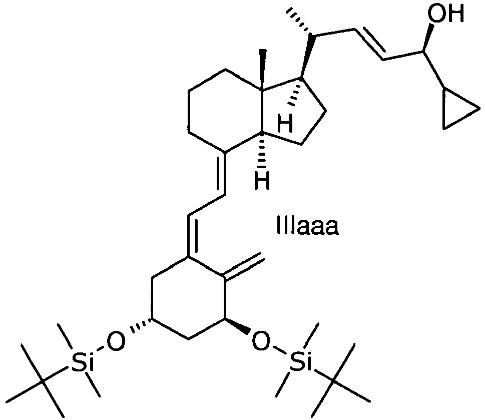 the method comprising the irradiation of a solution of a vitamin D derivative of general structure IIaaa, with a suitable light source in the presence of a triplet sensitizer with a triplet energy in the range of 150-270 kJ/mol;
wherein said solution is moving in multiple pass continuous flow relatively to the light source in a flow-through photoreactor or continuous flow photoreactor, characterised in that a fraction of the total solution is continuously and repeatedly circulated from a reservoir through the flow-through photoreactor or continuous flow photoreactor back to the reservoir.

In yet another aspect, this invention relates to the use of a flow-through photoreactor or continuous flow photoreactor in the manufacture of calcipotriol or calcipotriol hydrate.

In yet another aspect, this invention relates to a method for the manufacture of a pharmaceutical formulation or medicament containing calcipotriol or calcipotriol monohydrate, such as a cream, an ointment or a gel comprising a method as above.

### BRIEF DESCRIPTION OF THE DRAWINGS

Figure 1 is a longitudinal cross-section of an example of a suitable flow-through photoreactor or continuous flow photoreactor according to the present invention.
Figure 2 is a transverse cross-section taken through the dotted line of the example of the photoreactor depicted in Figure 1.

### DETAILED DESCRIPTION OF THE INVENTION

The previously described photoisomerisation processes for IIaaa have a number of disadvantages, especially on an industrial scale, such as the requirement of a high photocatalyst load and the fact that the reaction is run rather dilute and thus requires large volumes of solvent.

In industrial chemistry high substrate concentrations are usually preferred because of the cost of solvent and volume of production equipment. However, the use of highly concentrated reaction solutions in photochemistry is not straightforward. Synthetic organic photochemistry is usually performed in solution using immersion well reactors. These are most commonly fixed volume batch reactors irradiated from within inside using a single mercury vapour discharge lamp. These types of batch apparatus have limited application for large-scale photochemical synthesis as the amount of solution that can be effectively irradiated by the light source is scale dependent since the majority of the photochemistry only occurs within a short radius of the lamp. High concentrations of the light-absorbing substances may further reduce the thickness of the photoreaction zone (a photocatalytic reaction only proceeds on a surface of light-irradiated photocatalyst) and reduce the uniformity of the fluid's exposure to photons radiating from the light source. Concentrated solutions in a batch process may promote side reactions and as a consequence, many photoreactions must be performed in dilute solutions.

Moreover conventional batch photoisomerisation methods, e.g. of compound IIaaa, usually yield a mixture containing unreacted starting material, e.g. IIaaa, and often inevitably contain a significant amount of undesired degradation products, e.g. compounds of general structure IV, which then have tediously to be removed by chromatography.

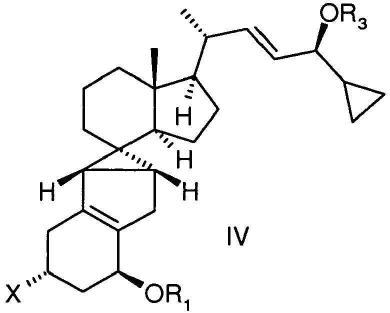

In general, the relationship between a suitable reactor design and the requirements for a specific photochemical reaction are still not fully understood. The choice of a specific photochemistry set-up and suitable reaction conditions, such e.g. substrate and photocatalyst concentration, irradiation time, and reactor design, is thus still unpredictable and remains a challenge, especially on an industrial scale.

### Definitions

As used herein a "hydroxy protecting group" includes any group which forms a derivative that is stable to the projected reactions wherein said hydroxy protecting group can be selectively removed by reagents that do not attack the regenerated hydroxy group. Said derivative can be obtained by selective reaction of a hydroxy protecting agent with a hydroxy group. Silyl derivatives, such as *tert*-butyldimethylsilyl forming silyl ethers are examples of hydroxy protecting groups. Silyl chlorides such as *tert*-butyldimethylsilyl chloride (TBSCI), trimethylsilylchloride, triethylsilylchloride, diphenylmethylsilylchloride, triisopropylsilylchloride, and *tert*-butyldiphenylsilylchloride are examples of hydroxy protecting agents. Hydrogen fluoride, such as aqueous HF in acetonitrile, or tetra n-butylammonium fluoride are examples of reagents which can remove silyl groups. Other hydroxy protecting groups include ethers, such as tetrahydropyranyl (THP) ether, including alkoxyalkyl ethers (acetals), such as methoxymethyl (MOM) ether, or benzyl ether, or esters, such as chloroacetate ester, trimethylacetate, acetate or benzoate ester. Non-limiting examples of hydroxy protecting groups and methods of protection and removal, all included in the scope of this application, can for example be found in "Protective Groups in Organic Synthesis", 3rd ed., T. W. Greene & P. G. M. Wuts eds., John Wiley 1999 and in "Protecting Groups", 1st ed., P.J. Kocienski, G. Thieme 2000, all of which are hereby incorporated by reference.

In the present context, the term "alkyl" is intended to indicate the radical obtained when one hydrogen atom is removed from a hydrocarbon. Said alkyl comprises 1-20, preferably 1-12, such as 1-7, such as 1-4 carbon atoms. The term includes the subclasses normal alkyl (*n*-alkyl), secondary and tertiary alkyl, such as methyl, ethyl, *n-*propyl, isopropyl, *n*-butyl, isobutyl, *sec*.-butyl, *tert*.-butyl, pentyl, isopentyl, hexyl, isohexyl, and the *tert*-butyldimethyl group.

In the present context, the term "substantially dissolved" is intended to indicate that the vitamin D derivatives in the *E* or *Z* form or as mixtures thereof may be either completely dissolved or they may be partially dissolved, such as in suspension, emulsion. The term "solution" includes substantially dissolved substrates.

### Embodiments

A suitable photochemical reactor for the present invention may be any reactor usually used in photochemistry which is suitable or adapted for flow-through, e.g. continuous flow. Such reactors are well-known to a person skilled in the art of photochemistry and can for example be found in "Ullmann's Encyclopeia of Industrial Chemistry, Photochemistry, A19, pp. 576-582 and in Vol B4 page 116-120" or in "International Chemical Engineering, Vol 12, No.1., 1972, pp. 131-143". Examples of photoreactors include, but are not limited to a tubular reactor, a bubble column reactor, a stirred tank reactor, a falling film reactor, or a belt reactor, all of which may be adapted for flow-through or continuous flow. The reactor may be used in series or parallel including various combinations of different reactors. More generally a suitable flow-through photoreactor or continuous flow photoreactor reactor may include a reactor body that circumscribes a longitudinally extending channel having a generally annular cross-section which, for example, accommodates fluids passing between an inner wall of the reactor body and an outer wall of a photon transmitting tube which, for example, is housed in an internal portion of the reactor and arranged in essentially co-axial alignment (i.e. longitudinally centered and in concentric relation) relative to the inner wall of the reactor. Another example of a suitable photoreactor is an in-line reactor with a generally cylindrical inner wall wherein the light housing tube is centered in co-axial relation therewith. The photoreactor may include mechanically static, fluid dynamic elements for passively inducing turbulent flow within a fluid passes through the channel, such as described in WO 96/35508 and references cited therein which are hereby incorporated by reference.

In one or more embodiments of the present invention, the flow-through photoreactor or continuous flow photoreactor reactor is an essentially axi-symmetrical tubular flow reactor wherein the solution is moving parallel to the central longitudinal axis. In one or more embodiments of the present invention, the essentially axi-symmetrical tubular flow reactor comprises at least two concentric tubular spaces coaxially aligned, such as longitudinally extending cylinders or tubes located one inside another, e.g. wherein an inner tubular space provides a light permeable housing for a light source, and wherein an outer tubular space provides a reaction chamber. In yet another embodiment of the present invention, the essentially axi-symmetrical tubular flow reactor comprises at least three concentric tubular spaces, e.g. three concentric cylinders or tubes located one inside another, wherein the inner tube representing a first tubular space provides the housing for the light source and wherein the second tubular space provides the reaction chamber, and wherein the third tubular space is adapted to be used as a cooling mantle. The irradiation volume aligned with the central axis may for example be of a length of about 5 to about 100cm, e.g. 50-70cm, such as 60cm.

In one or more embodiments the present invention relates to the use of a flow-through photoreactor or continuous flow photoreactor wherein a solution of a vitamin D-analogue is moving in single pass or circulating in multiple pass continuous flow relatively to the light source. This allows the photoisomerisation to be carried out in a convenient large-scale production process and has a number of advantages. This operation mode also may allow controlling the light irradiation by tuning the light-contact through flow adjustment. Furthermore the flow may be interrupted and resumed whenever convenient, e.g. in connection with a lamp exchange or reparation. Unlike a fixed batch process, the efficiency of the process may become scale independent. The continuous flow reactor may produce any desired throughput of feedstock by flowing for longer periods of time and without redesigning the reactor for larger quantities of product. Hence, larger volumes may be isomerised using a relatively smaller flow-through photoreactor or continuous flow photoreactor compared to a fixed batch reactor. In one or more embodiments of the present invention the solution of the vitamin D-analogue may be multiply collected and re-circulated through the flow-through photochemical reactor, e.g. a fraction of the total solution may be continuously and repeatedly circulated from a reservoir through the flow-through photochemical reactor back to the reservoir. The circulation of the solution through the flow-through photoreactor or continuous flow photoreactor, e.g. in conjunction with one or more reservoirs allows a great operational flexibility in a manufacturing plant. E.g. a single photoreactor may be used in connection with production in one or more batch reactors (reservoirs) of varying sizes, e.g. by connecting in series or parallel one or more photoreactor(s) to one or more reservoir units, which optionally also may be connected in series or parallel, via hoses or pipes. A specific photoreactor may be for example be fed from any remote chemical reactor or reservoir, e.g. by means of appropriate tubes, pipes or hoses.

Furthermore, the irradiation time (residence time) which is mainly defined by the flow rate may be easily controlled or operationally adjusted on a batch-to-batch basis by using in-process controls. Hence, by adjusting the flow rate or the re-circulation rate, the contact time of the solution with the light source (photon dose) may be tuned. Batch-to-batch variations or a fading of the lamp may thus be compensated or corrected and the risk of degradation due to over-radiation may be reduced. In one or more embodiments of the present invention the flow-rate is such that the flow of the reaction mixture in the photoreaction chamber is turbulent. Suitable flow rates which will, among other factors, depend on the design and dimension of the process equipment may for example be in the range of 2 l/min to 200 l/min, such as 3.6 l/min to 100 l/min, such as 4.8 l/min to 70 l/min, such as 10 l/min to 65 l/min, e.g. 40 l/min, 41 l/min, 42 l/min, 43 l/min, 44 l/min, 45 l/min, 46 l/min, 47 l/min, 47.1 l/min, 47.2 l/min, 47.3 l/min, 47.4 l/min, 47.5 l/min, 47.6 l/min, 47.7 l/min, 47.8 l/min, 47.9 l/min, 48 l/min, 48 l /min, 48.1 l/min, 48.2 l/min, 48.3 l/min, 48.4 l/min, 48.5 l/min, 48.6 l/min, 48.7 l /min, 48.8 l/min, 48.9 l/min, 49 l/min, 50 l/min, 51 l/min, 52 l/min, 53 l/min, 54 l /min, 55 l/min, 56 l/min, 57 l/min, 58 l/min, 59 l/min, or 60 l/min.

In one or more embodiments of the present invention, the solution is multiply collected and re-circulated through the flow-through photoreactor or continuous flow photoreactor.

In one or more embodiments of the present invention, a fraction of the total solution is continuously and repeatedly circulated from one or more reservoir(s) through a flow-through photoreactor or continuous flow photoreactor reactor back to the reservoir(s), wherein the solution is optionally mixed and temperature controlled in said reservoir(s). The percentage of the total solution present in the photoreactor and actually being irradiated, e.g. to be found in the photoreactor, may typically vary between 0.5%-99% of the total solution, e.g. 1-35 % of the total solution, such as 2-30%, e.g. 3-25%, e.g. 4-20%, e.g. 5-10%, e.g. 6-7%. In one or more embodiments of the present invention the irradiation volume of the photoreactor is about 101 and the volume of the reservoir is about 1701.

Any light source or lamp, including a plurality of lamps, which provide a spectral range and intensity appropriate to the photocatalyst and substrate used, optionally combined with a suitable cut-off filter, may be used in the present invention. Accordingly, the term light source includes a lamp combined with a suitable cut-off filter. The light sources may be of various geometries and are advantageously adapted to the geometry of the housing and/or reaction chamber, e.g. extended light sources. Suitable light sources can for example be found in "Ullmann's Encyclopaedia of Industrial Chemistry, Photochemistry, A19, pp. 576-582. In one or more embodiments, the light source provides polychromatic light, including UV light, such as in the range of 230-400 nm, e.g. 270-350, 300-340, 290-320 nm, 300-315 nm, or 310-312 nm. Suitable light sources are commercially available from various suppliers such as Heraeus, Hanau, or Günther H. Peschl (Bodenheim, Germany). In one or more embodiments of the present invention the light source comprises a mercury lamp, e.g. a high pressure lamp, or low pressure lamp, and in particular a medium pressure mercury lamp. The medium or high pressure mercury lamp may be doped with another metal such as antimony, bismuth, indium, thallium, or iron. The medium pressure mercury lamp may for example be operated with an electrical power input of about 2-60 kW, e.g. 3-20 kW or 3.4-10 kW, e.g. 3-7 kW, such as 6 kW. More specifically the lamp may, for example, be a TQ 718 Hanau lamp, a Günther H. Peschl Z0, Z2, or Z4 lamp, or lamps with similar like photon emitting characteristics. The lamp may typically have a length of about 5 to about 100cm, e.g. about 50 to about 70cm, such as about 55 to about 65 cm, e.g. 60cm.

The light source may be applied internally or from an inside portion of the reaction chamber, such as of being inside of two concentric tubes surrounding the lamp and defining the irradiation volume, or by being immersed in the reaction solution. The reaction mixture may also be irradiated from outside the reaction chamber, e.g. by using a focussing reflector or by using multiple lamps, e.g. a Rayonet-type apparatus. The present invention includes all embodiments where for example a plurality of light sources or lamps, same or different, are used, including all embodiments where the light sources are placed in various positions relatively to the reaction chamber.

In one or more embodiments of the present invention, the photochemical reactor comprises a housing for a light source. One of the advantages of having said housing for the light source is that it may allow an easy access and replacement of the light source. In one or more embodiments of the present invention the lamp-housing has an opening at one longitudinal end portion adapted to reversibly insert a lamp into the lamp-housing while allowing the reactor flow to continue. This allows to insert the lamp without the need to re-assemble the whole photoreactor, an advantage especially on a production scale where the lamp may be replaced without interrupting the circulation flow. Furthermore the housing, provided it is fitted with suitable cooling means, may allow the cooling of the light source. For example, the light source may be cooled by a cooling fluid or gas flowing through or around the housing, such as water. For example may water flow between the inner and outer wall surrounding the lamp. Alternatively the lamp may already be fitted with a cooling system. The geometry of the light source may advantageously be fitted to the housing. In order to allow the light generated by the light source to reach the irradiation volume, the housing may comprise a light permeable wall surrounding the light source, such as a wall made of quartz or boron silicate glass. In one or more embodiments of the present invention, the lamp is cooled inside the lamp housing by a flow of inert gas such as nitrogen and additionally the lamp housing is cooled from the outside portion by a cooling fluid, such as water.

In one or more embodiments of the present invention, the shortest distance in the irradiation volume traversable by the light emitting in vertical or perpendicular direction from the surface of the light source or the surface of the housing of the light source, or the average diameter of the reaction chamber, or the spacing between the tubular walls respectively, is less than about 30 cm, such as less than about 25, 20, 19, 18, 17, 16, 15, 14, 13, 12, 11, 10, 9.7, 9, 8, 7, 6, 5, 4, or 3 cm, e.g. 2.5, 2.0, 1.5, 1.0, 0.9, 0.85, 0.8, 0.75, 0.6, 0.5, 0.4, 0.3, 0.2cm, or 0.15cm. In one or more embodiments of the present invention the reaction chamber is defined by a spacing between the inner boundary tube and the outer boundary tube by about 2 mm to about 15 cm, such as about 25, 20, 19, 18, 17, 16, 15, 14, 13, 12, 11, 10, 9.7, 9, 8, 7, 6, 5, 4, or 3 cm, e.g. 2.5, 2.0, 1.5, 1.0, 0.9, 0.85, 0.8, 0.75, 0.6, 0.5, 0.4, 0.3, 0.2cm, or 0.15cm.

In one or more embodiments of the present invention, the photoisomerisation is carried out under essentially oxygen free conditions. The presence of oxygen in the reaction mixture might lead to the formation of singlet oxygen which can destructively react with the vitamin D derivatives. Essentially oxygen free conditions may be achieved by carrying the isomerisation out under an inert atmosphere, such as under an argon, helium or SF₆ atmosphere, preferably a nitrogen atmosphere. All reagents and solvents may be degassed and or the reaction chamber be evacuated and purged with an inert gas before irradiation to reduce the concentration of oxygen.

Non-limiting examples of photoreactors and light sources and combinations thereof can be for example found in US 5,012,106, US 3,554,887, US 4,456,512, DE 3625006,DE 10236717, EP 0000773, US 4,087,342, US 4,454,835, J. Org. Chem. 2005, 70, 7558-7564, US 5,126,111, US 4,296,066, Adv. in Photochemistry Vol. 18, 235-313, 1993, all of which are hereby incorporated by reference.

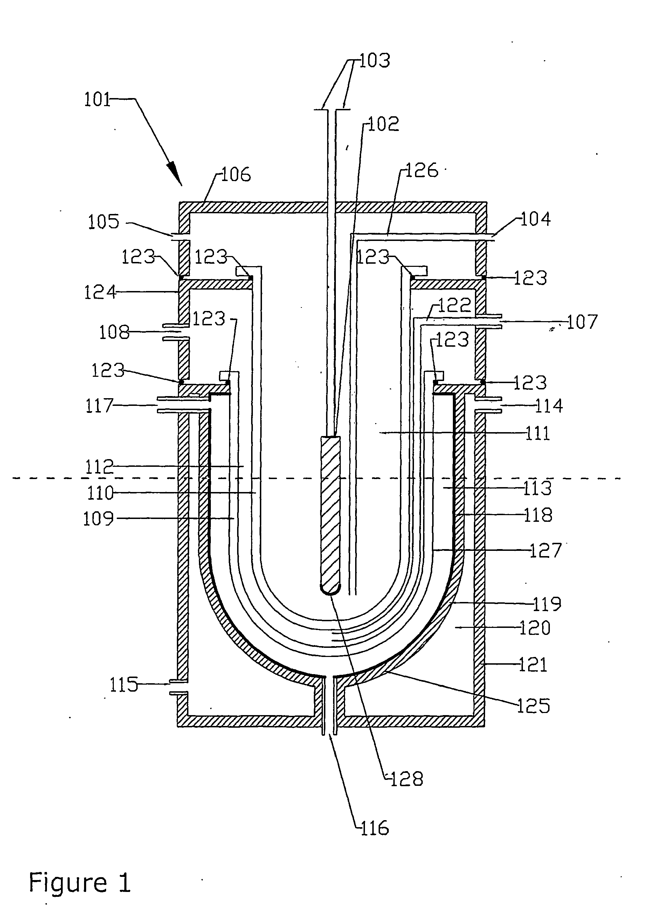

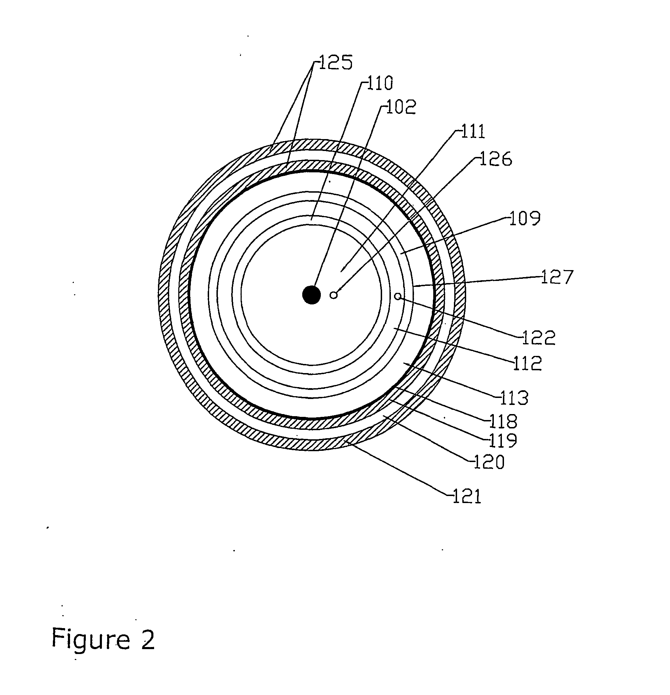

It is believed that the invention will be better understood in conjunction with the accompanying drawings illustrated in Figure 1 and Figure 2, which illustrate a non-limiting example of a photoreactor suitable for carrying out the present invention.

The photoreactor 101 comprises a reaction vessel 125, an outer light source housing 109, an inner light source housing 110, a light source or lamp 102, a top cover 106, and a connecting member 124.

Reaction vessel 125, outer light source housing 109, inner light source housing 110, top cover 106, light source 102, and connecting member 124 are all adapted to be fitted with connecting means 123, which allows them to be coupled together in concentric relationship. Suitable connecting means include, but are not limited to a connector or fastener such as a bolt and a nut, a joint, a clip, a clamp, a screw, or combinations thereof.

Outer light source housing 109 is adapted for seating in reaction vessel 125 with connecting means 123, connecting member 124 is adapted for seating on the reaction vessel 125 with connecting means 123, inner light source housing 110 is adapted for seating in connecting member 124 with connecting means 123, and the top cover 106 is adapted for seating on connecting member 124 with connecting means 123.

The inner light source housing 110, the top cover 106, and the connecting member 124 define a volume of an inner light source compartment 111, adapted to house light source 102. The inner light source compartment 111 is provided with gas supply means, such as a gas inlet 104 and a gas outlet 105 located in the top cover 106 for diffusion of gas through said compartment 111. The inner light source compartment 111 is further provided with means for electrical power supply 103 of light source 102.

The inner light source housing 110, the connecting member 124, and the outer light source housing 109, and the reaction vessel 125 define a volume of an outer light source compartment 112, adapted to provide cooling means to temperate the heat generated by light source 102, such as a cooling liquid or gas inlet 107 and a cooling liquid or gas outlet 108 located in the connecting member 124 for diffusion of cooling liquid or gas, e.g. water through said compartment 112.

Preferably, the gas supply and cooling liquid supply means comprise inlet tubes 122 or 126 for cooling liquid or gas supply respectively, wherein the tube endings are located at a bottom part of the light source compartments 111 and 112 respectively, and wherein gas or cooling liquid outlets 105 and 108 are located at an upper portion of the light source compartments 111 and 112 respectively.

The reaction vessel 125 comprises an outer wall of a cooling mantle 121 and an inner wall of the reaction chamber 119 which define a volume of a double walled cooling mantle 120. The reaction vessel 125 is provided with cooling means, e.g. the outer wall of the cooling mantle 121 is provided with a cooling liquid inlet or outlet 114 and a cooling liquid outlet or inlet 115, which are preferably located at spatially separated portions of the cooling mantle, such as the cooling liquid outlet 115 is located at a bottom portion of the reaction vessel 125 and the cooling liquid inlet 114 is located at an upper portion of the reaction vessel 125, for circulation of cooling water to temperate the solution to be photoisomerised in reaction chamber 113 by removing heat generated by the light source 102. The reaction vessel 125 is further provided with substrate supply means, such as a substrate inlet 116 and a substrate outlet 117. Preferably, the substrate inlet 116 is located at a bottom portion of the reaction vessel 125 and the substrate outlet 117 is located at an upper portion of the reaction vessel 125. All inlets and outlets may optionally comprise valves and/or nozzles.

The outer light source housing 109 and the reaction vessel 125 define a centrosymmetric concentric reaction chamber 113, essentially defined by parallel spacing of the inner wall of the reaction chamber 119 and the outer surface of the outer light housing 109, adapted to hold the reactants for a given photochemical reaction. The outer light source housing 109, the reaction vessel 125, the inner light source housing 110, and the light source 102 may be fitted so that the light intensity within the reaction chamber 113 may be essentially equally distributed for equal distances emitting in vertical direction from the outer surface of the outer light housing 127.

The light source 102 may be optionally fitted with light barriers 128 at the lower end portion of said light source, which prevent the light generated by said light source from irradiating into vertical downward direction.

The inner surface of the inner wall of the reaction chamber 119 may be optionally fitted with a coating 118 which is capable of absorbing light, such as black Teflon, capable of reducing light reflection.

Reaction chamber 113, cooling mantle 120, outer light source compartment 112, and inner light source compartment 111, may be seen to comprise a series of concentric cylinders or tubes nested one inside the other.

The housing of the light source and the reaction chamber are preferably substantially made of light-transmitting quartz or glass. Generally non-metallic materials are preferred, such as poly(methyl methacrylate), standard window glass, Pyrex (Corning 774), Vycor 791, Suprasil I (Heraeus), Suprasil-W (Heraeus), borosilicate glass, such as borosilicate glass 3.3 (ISO 3585:1998). In one or more embodiments of the present invention both the housing of the light source substantially consist of quartz and the and the part or wall of the reaction chamber closest to the light source 109 substantially consists of borosilicate glass. Because of its transmittance and thermal properties, quartz is a preferred material for construction of the inner light source housing 110. In one of more embodiments of the present invention, the material for the outer light housing 109 is borosilicate glass 3.3 (ISO 3585:1998 and EN 1595). A suitable materiel for the reaction vessel 125, the connecting member 124 and the top cover 106 is stainless steel.

In one specific embodiment of the present invention the present invention, the outer diameter of the inner light source housing 110 is about 61 mm, the inner diameter of the outer light source housing 109 is about 72 mm and the outer diameter is about 79mm corresponding to a wall thickness of the outer light source of about 3.5mm. In another specific embodiment of the present invention the present invention the outer light source housing 109 has a length of about 100 cm. In yet another specific embodiment of the present invention the inner diameter of the tubular space defined by the inner wall 119 of the reaction chamber is about 95 mm resulting in an irradiation layer thickness of about 8mm. In yet another specific embodiment of the present invention the lamp 102 has a length of about 60 cm and the bottom end said light source is positioned about 10 cm above the bottom of the inner light source housing 110, wherein said light source housing 110 for example has a length of about 130 cm.

Suitable photocatalysts are, for example, triplet sensitizers with a triplet energy in the range of 150-270 kJ/mol, e.g. below 185kJ, such as 170-180 kJ/mol, e.g. 176-178 kJ/mol. The E/Z ratio of the isomerisation at equilibrium can be tuned by choosing an appropriate energy of the triplet sensitizer. Such photocatalysts include, but are not limited to anthracene, 9-acetylanthracene, anthracene-9-carboxylic acid, anthracene-carboxaldehyde, phenazine, anthracene-9-sulfonic acid, 4,4-bis(dimethoxy)thiobenzophenon, 4,4-bis(dimethylamino)benzophenone, 4,4-bis(dimethylamino)thiobenzophenon, 4,4-bis(dimethoxy)benzophenone, or 9,10-diphenylanthracene. The photocatalysts may be used as mixtures but are preferably used as single compounds. In one or more embodiments of the present invention, the photocatalyst is present at a molar ratio of about 0.08 - 0.35 mole photocatalyst / mole vitamin D derivative, such as of about 0.1-0.2 mole photocatalyst / mole vitamin D derivative, e.g. 0.15-0.18 photocatalyst / mole vitamin D derivative, e.g. 0.175 photocatalyst / mole vitamin D derivative.

Suitable solvents include any solvent or solvent mixture which is capable of at least partially dissolving the vitamin D derivatives and the photosensitizer, which essentially do not interfere with the reaction conditions and which do not absorb significantly the light generated from the light source in the spectral range which is required for the photoreaction. Suitable solvents include halogenated hydrocarbons, such as dichloromethane, ethers, such as tert-butylmethyl ether (MTBE), tetrahydrofuran, dioxane, dimethoxyethane, hydrocarbons such as hexane, heptane, toluene, and triethylamine, or mixtures thereof. The addition of traces of bases, such as triethylamine may be advantageous since vitamin D derivatives usually are acid sensitive. Preferred solvents are dichloromethane or MTBE. Especially when 9-acetylantracene is use as the photocatalyst, the solvent is advantageously MTBE optionally comprising triethylamine. The present invention includes mixtures of said solvents of all compositions. Electrostatic charging may be avoided by adding protic solvents, e.g. tert-butanol, which do not participate in the photoreaction to the solvents.

In one or more embodiments of the present invention the vitamin D derivatives of general structure II are dissolved in the solvent at or above a concentration in the range of about 0.003 g - about 0.0134 g / ml solvent, such as about 0.025 g - about 0.1 g /ml solvent, such as about 0.04 g - about 0.06 g / ml solvent, e.g. 0.05 g / ml solvent.

The photoisomerisation may be carried out at a temperature range of about -20-50°C, - 10-40°C, such as 0-30 °C, such as 5-25 °C, e.g. at a temperature of about 10-15°C, such as at about 10, 11, 12, 13, 14, or 15°C.

In order to optimise the yield of the desired photoisomerisation product, the irradiation may be carried out until at least 80% , e.g. 90%, such as 91%, 92%, 93%, 94%, 95%, 96&, 97%, 97.5%, 98%, 98.5%, 99%, 99.5%, 99.6%, 99.7%, 99.8%, 99.9% of the vitamin D derivate of general structure IIa, IIb, IIc, or IId are isomerised to IIIa, IIIb, IIIc, or IIId respectively. The light irradiation may usually be carried out for about 1-25 hours, such as 6-20 hours, or 7-10 hours, or 5-8 hours, depending on the specific reaction conditions used, the irradiation may be longer or shorter.

Any numeric interval described in this application shall include any specific number, or narrower interval, lying within that interval as a more specific embodiment of the present invention. The term "about" is, supplementary to its common meaning, intended to indicate the inclusion of a numerical interval which lies 10% below or above the value or number related to.

The photoisomerisation methods described herein may be useful in the synthesis of other vitamin D derivatives, such as for the isomerisation of intermediates useful for the synthesis of calcitriol or alpha-calcidol, e.g. the isomerisation of the 5-E calcitriol precursor to 5-Z calcitriol, wherein one or more hydroxy groups may be protected, e.g. by tert-butyldimethylsilyl, or unprotected; or the isomerisation of 5-*E* di-hydroxy protected alpha-calcidol precursor to 5-Z di-hydroxy protected alpha-calcidol, e.g. the isomerisation of (1a,3b,5E,7E)-9,10-secochola-5,7,10(19)-triene-1,3-bis(((1,1-dimethylethyl)dimethylsilyl)oxy to the corresponding 5Z-isomer followed by removal of the protecting groups to give (5Z,7E)-9,10-secocholesta-5,7,10(19)-triene-la,3b-diol.

### Synthetic Methods

Compounds of general structure IIa, IIb, IIc, IId, or IIe can for example be synthesised according to methods disclosed for example by M. J. Calverley, Tetrahedron, Vol. 43, No. 20, pp. 4609-4619, 1987, in WO 87/00834, WO 2005/095336, WO 2005/087719, US 5,763, 426, WO 03/106412, or in Drugs of the Future Vol 15., No.1, 1990, page 15, or in Bioorg. Chem. Lett. Vol 3 No. 9 1841-184, 1993. The same references also disclose methods of transforming compounds of general structure IIIa, IIIb, IIIc, IIId, or IIIe to calcipotriol or intermediates useful for the synthesis of calcipotriol.

General synthetic methods for vitamin D derivatives, such as compounds of general structure IIa, IIb, IIc, IId or IIe can also be found in ["Vitamin D", D. Feldman, Ed., Academic Press, San Diego, USA, 1997] and [G.-D. Zhu et al., Chem. Rev. 1995, 95, 1877-1952] and references cited therein. Pharmaceutical formulations or calcipotriol or calcipotriol hydrate, such as creams, ointments, solutions, lotions, etc., can be found in US 6,753,013, US 5,763,426, US 6,787,529, and US 4,866,048, or may be prepared by any of the methods known in the art, such as described in Lawrence H. Block, Medicated Applications, in , II Remington: The Science and Practice of Pharmacy 1577, 1585-91 (19thed., Alfonso R. Gennaro, ed., 1995).

The methods for producing calcipotriol or its monohydrate as described herein may be modified with regard to the order of the reaction steps, by omitting one or more reaction steps, or by introducing additional purification or reaction steps at any stage of the reaction sequence. The present invention includes all such modifications. The method for producing calcipotriol as described herein includes further all variants, where the hydroxy protecting groups R₁ and/or R₂ and/or R₃ for compounds or intermediates, where R₁ and/or R₂ and/or R₃ are not hydrogen, are removed or replaced by one or more other protecting groups at any stage of the reaction sequence. Compounds or intermediates, wherein R₁ and/or R₂ and/or R₃ are hydrogen may be protected with protecting agents at any stage of the reaction sequence, including protecting agents which yield other protecting groups than those removed earlier in the reaction sequence. The present invention relates to all isomeric forms either in pure form or as mixtures thereof. The indication of a specific conformation or configuration either in the formulas or the names of compounds or intermediates of the present invention shall indicate that this specific conformation or configuration is a preferred embodiment of the invention. The indication of a specific conformation or configuration either in the formulas or the names of compounds or intermediates of the present invention shall include any other isomer than specifically indicated, either in pure form or as mixtures thereof, as another embodiment of the present invention. Pure stereoisomeric forms of the compounds and the intermediates of this invention may be obtained by the application of procedures known in the art, such as by chromatography or crystallisation, or by stereoselective synthesis.

Methods for the crystallization of e.g. vitamin D derivatives, and in particular calcipotriol can for example be found in M. J. Calverley, Tetrahedron, Vol. 43, No. 20, pp. 4609-4619, 1987, WO 94/15912; WO 2004/046097 and include crystallization from mixtures of ethylacetate and hexane or heptane of suitable polarity. Calcipotriol hydrate may be obtained by crystallisation of calcipotriol from mixtures of organic solvents and water, such as for example by methods described in WO 94/15912. Accordingly, the present invention relates to the use of a flow-through photoreactor or continuous flow photoreactor reactor in the manufacture of alpha-calcidol or calcitriol.

### EXAMPLES

### General:

All chemicals, unless otherwise noted were from commercial sources. Analytical HPLC was performed Merck-Hitachi equipment: pump:L-6200 or L-6000A, detector: L-4000, integrator: D-2500, Noise level 6, Sensitivity 10. Chromatography was performed on silica gel optionally using the flash technique. Preferably the silica used for chromatography was from Merck KGaA Germany: LiChroprep® Si60 (15-25µm). Ethyl acetate, dichloromethane, or appropriate mixtures of ethyl acetate, dichloromethane, methanol, and petroleum ether (40-60) or heptane were used as eluents unless otherwise noted. ¹H nuclear magnetic resonance (NMR) spectra (300 MHz) were recorded on a Bruker DRX instrument. Chemical shift values (δ) (in ppm) are quoted, unless otherwise specified; for deuteriochloroform solutions relative to internal tetramethylsilane (δ = 0.00) or chloroform (δ = 7.26) standard.

### Example 1:

### Continuous photoisomerisation using a flow-through photochemical reactor

7.5 kg of 1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIa: X = OR₂, R₁, R₂ = *tert*-butyldimethylsilyl, R₃ = hydrogen) which was prepared as described earlier by M. J. Calverley, Tetrahedron, Vol. 43, No. 20, pp. 4609-4619, 1987 or in WO 87/00834, and 45 g 9-acetylanthracene were dissolved in 150 l essentially oxygen free methyl-*tert-*butylether (MTBE) under a nitrogen atmosphere with stirring. The mixture was continuously pumped from a 180 l stirred batch reactor through a continuous flow photoreactor having the dimensions as described earlier in the specification comprising a medium pressure mercury lamp doped with iron (power input 6kW, UVH5822F-1, power supply/ballast unit: 10 kW Heraeus) and back to the batch reactor (flow rate ca. 48 l/min). The temperature of the reaction mixture was kept at about 10°C by cooling of the stirred batch reactor and the photoreactor. The circulation and light irradiation was stopped when less of 1.5% of the starting material could be detected by HPLC (eluent: n-heptan:EtOAc (100:2), flow: 3,0 ml/min, column: LiChrosorb Si60 5µm, UV detector at 270nm). The residual content of the photoreactor was transferred to the stirred batch reactor, the photoreactor was washed once with 10 l MTBE and the washing was transferred to the stirred batch reactor as well. The solvent was removed *in vacuo* to give 1(S),3(R)-bis(*tert*-butyl-dimethylsilyloxy)-20(R)-(3'-cyclopropyl-3(S)'-hydroxyprop-1'(*E*)-enyl)-9,10-secopregna-5(Z),7(*E*),10(19)-triene (IIa: X = OR₂, R₁, R₂ = *tert-*butyldimethylsilyl, R₃ = hydrogen) after chromatography in full accordance with the data described by M. J. Calverley in Tetrahedron, Vol. 43, No. 20, p. 4618, 1987 for compound 28.

### Example 2:

### Calcipotriol

1(S),3(R)-bis(*tert*-butyl-dimethylsilyloxy)-20(R)-(3'-cyclopropyl-3(S)'-hydroxyprop-1'(*E*)-enyl)-9,10-secopregna-5(Z),7(*E*),10(19)-triene (IIa: X = OR₂, R₁, R₂ = *tert-*butyldimethylsilyl, R₃= hydrogen) obtained from Example 1 is deprotected using tetrabutyl ammonium fluoride in tetrahydrofuran at 60°C followed by chromatography, as described earlier by M. J. Calverley, Tetrahedron, Vol. 43, No. 20, pp. 4609-4619, 1987 or in WO 87/00834. Crystallisation from ethylacetate/hexane containing a few drops of triethylamine gives calcipotriol in full accordance with the data described by M. J. Calverley in Tetrahedron, Vol. 43, No. 20, p. 4618, 1987 for compound 4.

### Example 3:

### Calcipotriol monohydrate

The calcipotriol obtainable as outlined in example 2 is crystallised from ethyl acetate /water as described in WO 94/15912 to give calcipotriol monohydrate in full accordance with the characteristic data described in that patent.

### Example 4:

### Stirred batchreactor-photoisomerisation vs. continuous flow photosiomerisation

An oxygen-free solution of 1(S),3(R)-bis(*tert*-butyl-dimethylsilyloxy)-20(R)-(3'-cyclopropyl-3(S)'-hydroxyprop-1'(*E*)-enyl)-9,10-secopregna-5(*E*),7(*E*),10(19)-triene (IIaaa) in MTBE (1g/20ml) containing 450mg 9-acetylanthracene was photoisomerised in two different set-ups for direct comparison of batch vs. continuous flow. The photoreaction set-up was designed to allow both batch mode and continuous flow through the photoreactor parallel along the longitudinal axis of the reactor: In both set-ups the lamp providing UV-light (medium pressure mercury lamp doped with iron from Heraeus: TQ718 Z4, 800W, power supply Best. Nr. 56002316), which was surrounded by a quartz lamp-housing comprising inner and outer jackets for water cooling, was placed in the centre of a standard immersion well photoreactor adapted for stirred-batch and continuous flow-through operational mode by comprising closable inlet and outlet-openings. The inlet-opening of the photoreactor was placed on the bottom of the photoreactor and the outlet-opening on the top of the photoreactor just beneath the surface level when filled during operation to allow a flow in the reactor along the longitudinal axis of the immersed lamp when the pumping the solution. The lamp was temperature controlled by cooling the lamp housing with cooling water (10°C). The irradiation layer thickness of the photoreactor was 9.7mm. The reaction mixture was kept under nitrogen at all times.

### a) continuous flow mode:

In this set-up inlet- and outlet-openings of the photoreactor were connected via a pump through hoses to a reservoir. During pump operation, a fraction of the solution from the reservoir is pumped continuously and repeatedly circulated from the reservoir through the flow-through photochemical reactor back to the reservoir. Ca. 450 ml of the reaction mixture was moving inside the photoreactor while ca. 1050 ml of the reaction mixture were mainly in the reservoir (the remaining minor volume was circulating in the connecting hoses). The flow was controlled to be in the range of 3600ml/min to 4800 ml/min. The temperature of the reaction mixture was always kept at 10°C by cooling of the reservoir.

### b) stirred batch mode:

In this set-up the photoreactor is the same as above except that the inlet and outlet openings are both closed and the pump is not operated. The temperature of the reaction mixture was always kept at 10°C by cooling the photoreactor from outside. Furthermore, the 450ml reaction mixture volume was stirred by use of a bottom magnet and a magnetic stirrer to ensure vigorous agitation and circulation around the lamp inside the whole volume of the photoreactor. The mixing was ascertained to be effective in both vertically and horizontally direction by visual inspection.

The progress of the reaction was monitored by withdrawing samples at appropriate intervals and analysing them by HPLC (eluent: n-heptan:EtOAc (100:2), flow: 3,0 ml/min, column: LiChrosorb Si60 5µm, UV detector at 270nm). The same method was used for purity determination. The impurity content of general structure IV was determined by ¹H-NMR comparing the integration of the triene system of hydrogen 22/23 at 5.45 ppm vs. the integration of the sidechain hydrogen-24 at 3.42 ppm. The results of the experiments are illustrated in the following table:

| **Operational mode** | **a) continuous flow** | **b) stirred batch** |
|---|---|---|
| Irradiation time for complete conversion" | 54 min (for 75 g substrate) | 37 min (for 22.5 g substrate) |
| Irradiation time / substrate | 0.72 min / g | 1.64 min / g |
| Purity of IIIa^{#} (HPLC) | 80% | 65% |
| Impurity of general structure IV^{#} (NMR) | mol % | 15 mol % |

| | | |
|---|---|---|
| ¤ a):98.9%; b): 99.2% # (R₁ = tert-butyl-dimethylsilyl, X = tert-butyl-dimethylsilyloxy, R₃ = hydrogen) | | |

These results surprisingly indicate that the *E*-Z-photoisomerisation of IIaaa yields product IIIa^{#} in improved purity in a more efficient process with improved space-time-yield.

## Claims

1. A method of isomerising a solution of a vitamin D derivative of general structure IIa, IIb, IIc, IId or IIe respectively; 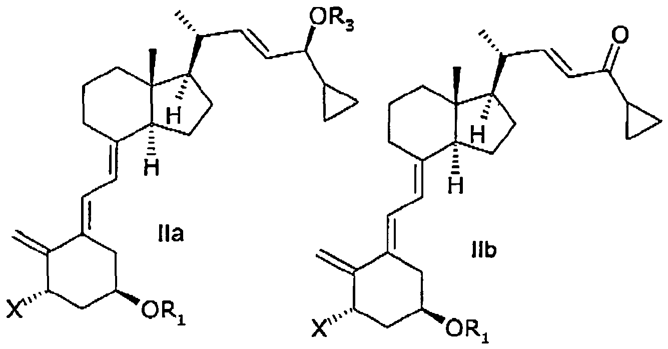 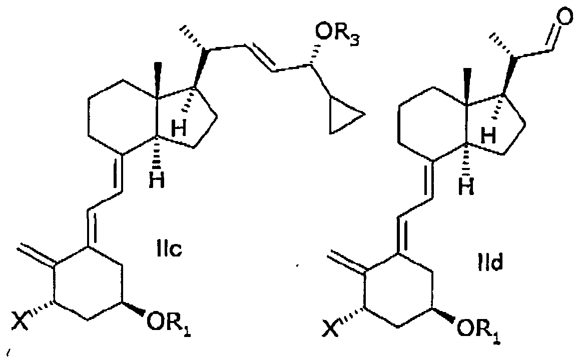 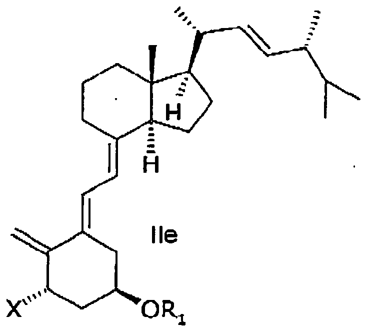 to give a vitamin D derivative of general structure IIIa, IIIb, IIIc, IIId or IIIe respectively; 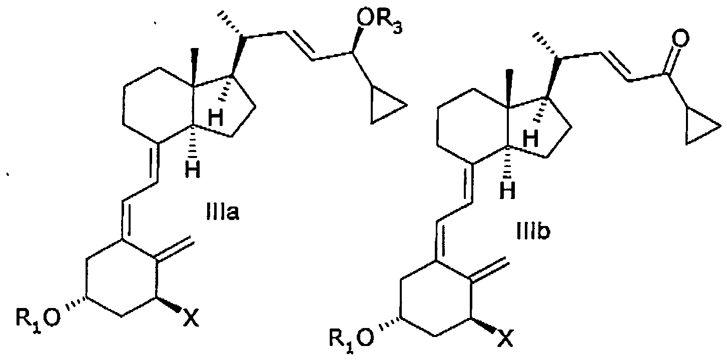 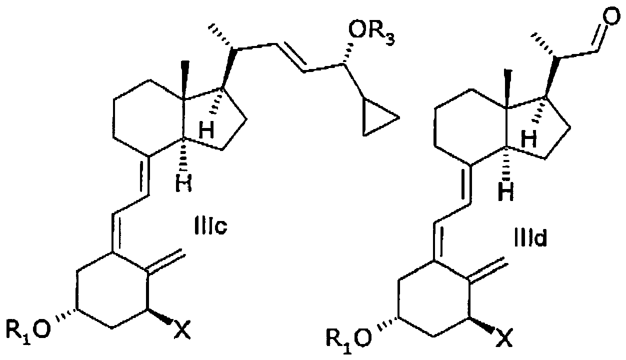 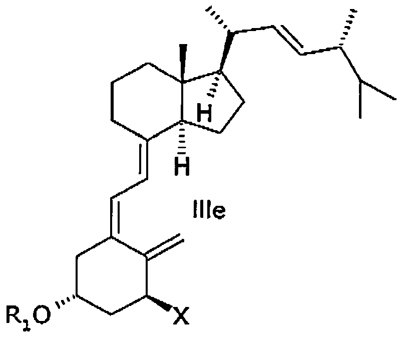 wherein X represents hydrogen or -OR₂;
R₁, R₂ and R₃ may be the same or different and independently represent hydrogen or a hydroxy protecting group;
the method comprising the irradiation of a solution of a vitamin D derivative of general structure IIa, IIb, IIc, IId or IIe respectively,
with a suitable light source in the presence of a triplet sensitizer with a triplet energy in the range of 150-270 kJ/mol in a flow-through photoreactor or continuous flow photoreactor, wherein said solution is moving in single pass or circulating in multiple pass continuous flow relatively to the light source in a flow-through photoreactor or continuous flow photoreactor reactor.

2. A method for producing calcipotriol {(5Z, 7E, 22E, 24S)-24-cyclopropyl-9,10-secochola-5,7,10(19),22-tetraene-1α-3β-24-triol} or calcipotriol monohydrate comprising the steps of
(i) isomerising a vitamin D derivative of general structure IIaa 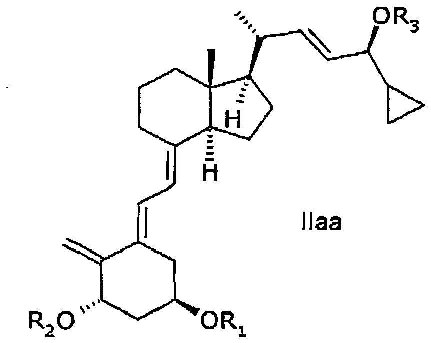 to give a vitamin D derivative of general structure IIIaa; 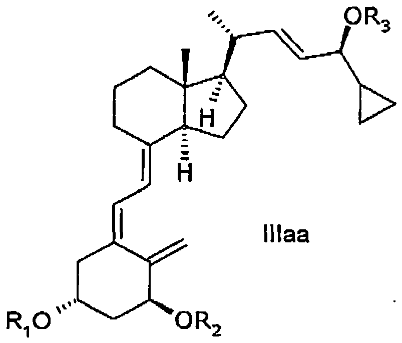 wherein R₁, R₂ and R₃ may be the same or different and independently represent hydrogen or a hydroxy protecting group;
with a suitable light source in the presence of a triplet sensitizer with a triplet energy in the range of 150-270 kJ/mol;
**characterised in that** said solution is moving in single pass or multiple pass continuous flow relatively to the light source in a flow-through photoreactor or continuous flow photoreactor;
(ii) when R₁ and/or R₂ and/or R₃ are not hydrogen, removing the hydroxy protecting group(s) R₁ and/or R₂ and/or R₃ of the compound of general structure IIIa to generate calcipotriol; and
(iv) optionally crystallising the calcipotriol from a mixture of an organic solvent and water to give calcipotriol monohydrate.

3. The method according to claim 1, wherein R₃ represents hydrogen and X represents - OR₂.

4. The method according to any one of the preceding claims, wherein R₁ and R₂ represent alkylsilyl or hydrogen.

5. The method according to any one of the preceding claims, wherein R₁ and R₂ represent *tert*-butyldimethylsilyl and R₃ represents hydrogen.

6. The method according to any one of the preceding claims, wherein the flow-through photoreactor or continuous flow photoreactor reactor is an essentially axi-symmetrical tubular flow reactor wherein the solution is moving parallel to the central longitudinal axis.

7. The method according to any one of the preceding claims, wherein the solution is multiply collected and re-circulated through the flow-through photoreactor or continuous flow photoreactor reactor.

8. The method according to any one of the preceding claims, wherein a fraction of the total solution is continuously and repeatedly circulated from one or more reservoir(s) through the flow-through photoreactor or continuous flow photoreactor reactor back to the reservoir(s), wherein the solution is optionally mixed and temperature controlled in said reservoir(s).

9. The method according to any one of the preceding claims, wherein the light source comprises a medium pressure mercury lamp doped with iron.

10. The method according to any one of the preceding claims, wherein the light source provides UV-light in particular in the range of 300 to 340 nm.

11. The method according to claim 9 or 10, wherein the medium pressure mercury lamp is operated with an electrical power input of 3 to 7 kW.

12. The method according to any one of the preceding claims, wherein the triplet sensitizer is selected from the group consisting of anthracene, 9-acetylanthracene, anthracene-9-carboxylic acid, anthracene-carboxaldehyde, phenazine, anthracene-9-sulfonic acid, 4,4-bis(dimethoxy)thiobenzophenon, 4,4-bis(dimethylamino)benzophenone, 4,4-bis(dimethylamino)thiobenzophenon, 4,4-bis(dimethoxy)benzophenone, and 9,10-diphenylanthracene, or mixtures thereof.

13. The method according to any one of the preceding claims, wherein the solvent is selected from the group consisting of dichloromethane, tert-butylmethyl ether, tetrahydrofuran, dioxane, dimethoxyethane, hexane, heptane, toluene, triethylamine, or mixtures thereof.

14. The method according to any one of the preceding claims, wherein the isomerisation is carried out at a temperature of 0 to 35°C under an inert atmosphere.

15. The method according to any one of claims 6-14, wherein the essentially axi-symmetrical tubular flow reactor comprises at least two concentric tubular spaces coaxially aligned, such as longitudinally extending cylinders or tubes located one inside another, wherein an inner tubular space provides a light permeable housing for a light source, and wherein an outer tubular space provides a reaction chamber.

16. The method according to claim 15, wherein the reaction chamber is defined by a spacing between the inner boundary tube and the outer boundary tube by 2 mm to 15 cm.

17. The method according to any one of the preceding claims, wherein the triplet sensitizer is present at a molar ratio of 0.08 to 0.35 mole triplet sensitizer / mole vitamin D derivative, and wherein the vitamin D derivatives are dissolved in the solvent at a concentration in the range of 0.025 g - 0.1 g / ml solvent.

18. A method of preparing calcipotriol or calcipotriol monohydrate comprising in one or more steps the method of any one of the preceding claims.

19. A method of isomerising a solution of a vitamin D derivative of general structure IIaaa; 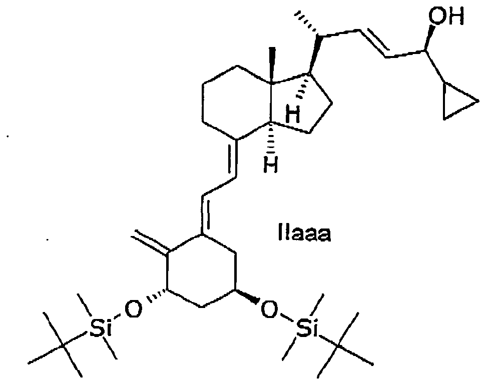 to give a vitamin D derivative of general structure IIIaaa, 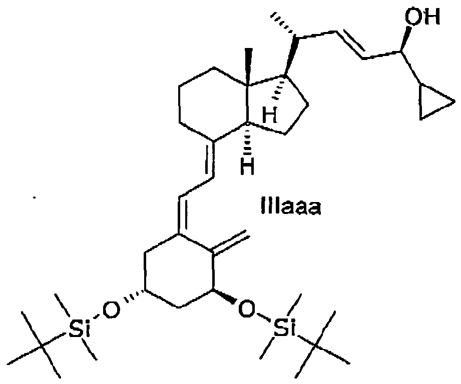 the method comprising the irradiation of a solution of a vitamin D derivative of general structure IIaaa, with a suitable light source in the presence of a triplet sensitizer with a triplet energy in the range of 150-270 kJ/mol;
wherein said solution is moving in multiple pass continuous flow relatively to the light source in a flow-through photoreactor or continuous flow photoreactor reactor; **characterised in that** a fraction of the total solution is continuously and repeatedly circulated from a reservoir through the flow-through photoreactor or continuous flow photoreactor reactor back to the reservoir.

20. A method for the manufacture of a pharmaceutical formulation or medicament containing calcipotriol or calcipotriol monohydrate, such as a cream, an ointment or a gel comprising the method according to any one of claims 1-19.

21. Use of a flow-through photoreactor or continuous flow photoreactor reactor in the manufacture of calcipotriol or calcipotriol hydrate.

22. Use of a flow-through photoreactor or continuous flow photoreactor reactor in the manufacture of alpha-calcidol or calcitriol.

23. The use according to claim 21 wherein the flow-through photoreactor or continuous flow photoreactor reactor is an essentially axi-symmetrical tubular flow reactor in which an elongated lamp-housing is essentially centrally placed in an inner portion of the reaction chamber and where said lamp-housing is longitudinally aligned with the flow direction.

## Patentansprüche

1. Verfahren zur Isomerisierung einer Lösung eines Vitamin-D-Derivates der allgemeinen Struktur IIa, IIb, IIc, IId bzw. IIe; 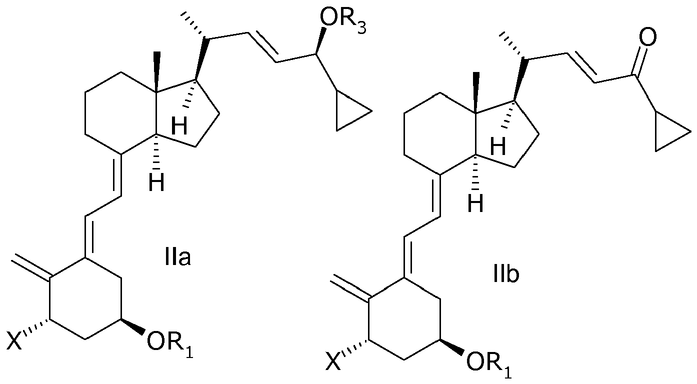 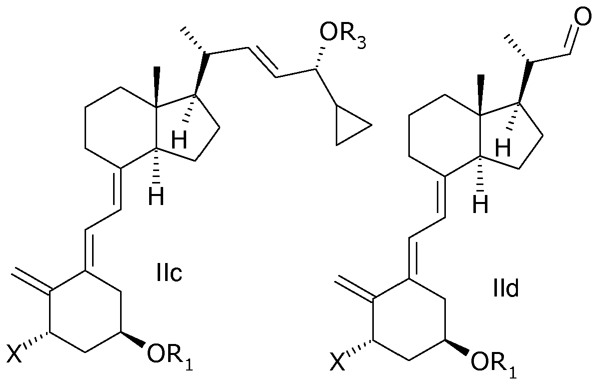 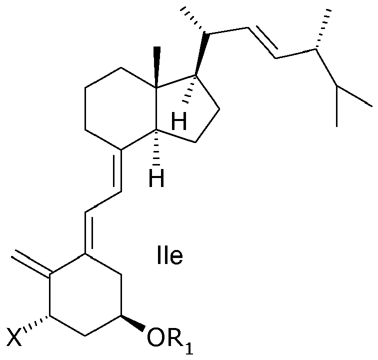 um ein Vitamin-D-Derivat der allgemeinen Struktur IIIa, IIIb, IIIc, IIId bzw. IIIe zu erhalten; 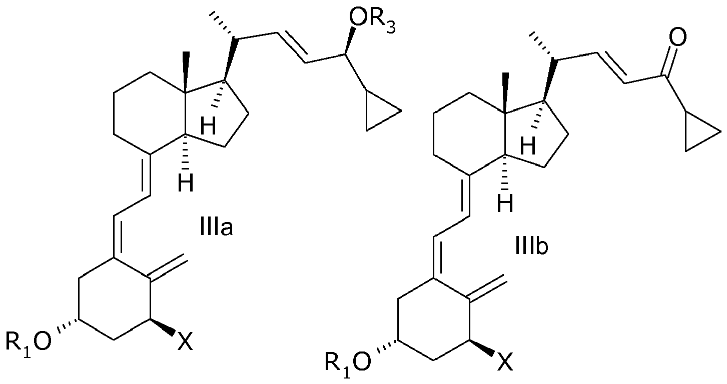 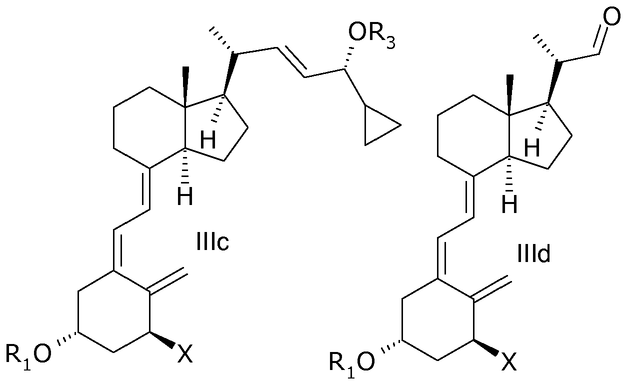 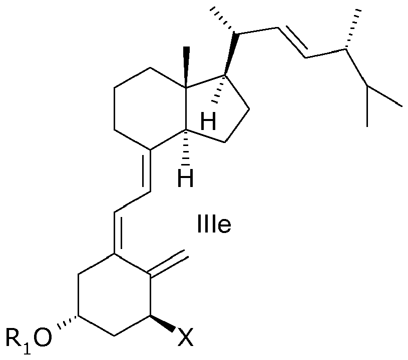 worin X für Wasserstoff oder -OR₂ steht;
R₁, R₂ und R₃ gleich oder unterschiedlich sein können und unabhängig voneinander für Wasserstoff oder eine Hydroxy-Schutzgruppe stehen;
wobei das Verfahren das Bestrahlen einer Lösung eines Vitamin-D-Derivates der allgemeinen Struktur IIa, IIb, IIc, IId bzw. IIe mit einer geeigneten Lichtquelle in Gegenwart eines Triplett-Sensitizers mit einer Triplett-Energie im Bereich von 150-270 kJ/mol in einem Durchfluss-Photoreaktor oder Photoreaktor mit kontinuierlicher Strömung umfasst, worin sich die Lösung in einem Durchlauf oder zirkulierend in mehreren Durchläufen mit kontinuierlicher Strömung relativ zu der Lichtquelle in einem Durchfluss-Photoreaktor oder Photoreaktor mit kontinuierlicher Strömung bewegt.

2. Verfahren zur Herstellung von Calcipotriol {(5Z,7E,22E,24S)-24-Cyclopropyl-9,10-secochola-5,7,10(19),22-tetraen-1α-3β-24-triol} oder Calcipotriolmonohydrat, wobei das Verfahren folgende Schritte umfasst:
(i) Isomerisieren des Vitamin-D-Derivats der allgemeinen Struktur IIaa, 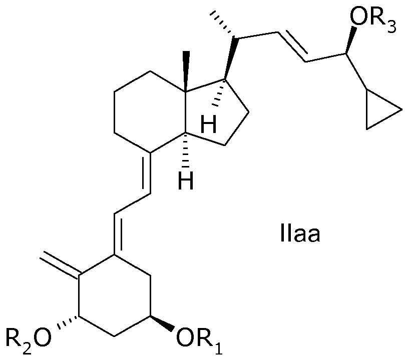 um das Vitamin-D-Derivat der allgemeinen Struktur IIIaa zu erhalten; 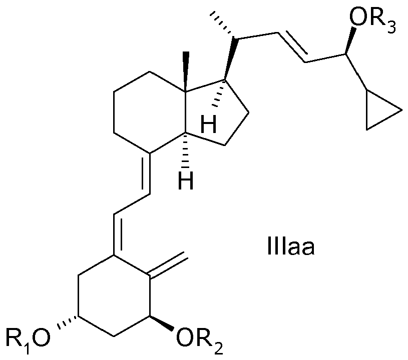 worin R₁, R₂ und R₃ gleich oder unterschiedlich sein können und unabhängig voneinander für Wasserstoff oder eine Hydroxy-Schutzgruppe stehen;
mit einer geeigneten Lichtquelle in Gegenwart eines Triplett-Sensitizers mit einer Triplett-Energie im Bereich von 150-270 kJ/mol;
**dadurch gekennzeichnet, daß** die Lösung sich in einem Durchlauf oder in mehreren Durchläufen mit kontinuierlicher Strömung relativ zu der Lichtquelle in einem Durchfluss-Photoreaktor oder Photoreaktor mit kontinuierlicher Strömung bewegt;
(ii) falls R₁ und/oder R₂ und/oder R₃ nicht für Wasserstoff stehen, Entfernen der Hydroxy-Schutzgruppe(n) R₁ und/oder R₂ und/oder R₃ der Verbindung der allgemeinen Struktur IIIa, um Calcipotriol zu erzeugen; und
(iv) gegebenenfalls Kristallisieren von Calcipotriol aus einem Gemisch eines organischen Lösungsmittels und Wasser, um Calcipotriolmonohydrat zu erhalten.

3. Verfahren nach Anspruch 1, wobei R₃ für Wasserstoff steht und X für -OR₂ steht.

4. Verfahren nach einem der vorhergehenden Ansprüche, wobei R₁ und R₂ für Alkylsilyl oder Wasserstoff stehen.

5. Verfahren nach einem der vorhergehenden Ansprüche, wobei R₁ und R₂ für *tert-*Butyldimethylsilyl stehen und R₃ für Wasserstoff steht.

6. Verfahren nach einem der vorhergehenden Ansprüche, wobei der Durchfluss-Photoreaktor oder Photoreaktor mit kontinuierlicher Strömung ein im Wesentlichen axial-symmetrischer Durchfluss-Rohrreaktor ist, in dem sich die Lösung parallel zur zentralen longitudinalen Achse bewegt.

7. Verfahren nach einem der vorhergehenden Ansprüche, wobei die Lösung mehrfach gesammelt wird und wiederholt durch den Durchfluss-Photoreaktor oder Photoreaktor mit kontinuierlicher Strömung zirkuliert.

8. Verfahren nach einem der vorhergehenden Ansprüche, wobei eine Fraktion der gesamten Lösung kontinuierlich und wiederholt von einem oder mehreren Reservoir(s) durch den Durchfluss-Photoreaktor oder Photoreaktor mit kontinuierlicher Strömung zurück zu dem/den Reservoir(s) zirkuliert, wobei die Lösung in dem/den Reservoir(s) gegebenenfalls durchmischt und die Temperatur gesteuert wird.

9. Verfahren nach einem der vorhergehenden Ansprüche, wobei die Lichtquelle eine Mitteldruck-Quecksilberlampe, welche mit Eisen dotiert ist, umfasst.

10. Verfahren nach einem der vorhergehenden Ansprüche, wobei die Lichtquelle UV-Licht insbesondere im Bereich von 300 bis 340 nm liefert.

11. Verfahren nach Anspruch 9 oder 10, wobei die Mitteldruck-Quecksilberlampe mit einer elektrischen Leistungsaufnahme von 3 bis 7 kW arbeitet.

12. Verfahren nach einem der vorhergehenden Ansprüche, wobei der Triplett-Sensitizer ausgewählt ist unter Anthracen, 9-Acetylanthracen, Anthracen-9-carbonsäure, Anthracen-carboxaldehyd, Phenazin, Anthracen-9-sulfonsäure, 4,4-Bis(dimethoxy)thiobenzophenon, 4,4-Bis(dimethylamino)benzophenon, 4,4-Bis(dimethylamino)thiobenzophenon, 4,4-Bis(dimethoxy)benzophenon und 9,10-Diphenylanthracen oder Gemischen davon.

13. Verfahren nach einem der vorhergehenden Ansprüche, wobei das Lösungsmittel ausgewählt ist unter Dichlormethan, tert-Butylmethylether, Tetrahydrofuran, Dioxan, Dimethoxyethan, Hexan, Heptan, Toluol, Triethylamin oder Gemischen davon.

14. Verfahren nach einem der vorhergehenden Ansprüche, wobei die Isomerisierung bei einer Temperatur von 0 bis 35 °C unter Inertatmosphäre durchgeführt wird.

15. Verfahren nach einem der Ansprüche 6-14, wobei der im Wesentliche axialsymmetrische Durchfluss-Rohrreaktor wenigstens zwei konzentrische Rohrbereiche umfasst, die koaxial ausgerichtet sind, etwa als longitudinal verlaufende Zylinder oder Röhren, die ineinander angeordnet sind, wobei ein innerer röhrenförmiger Bereich ein lichtdurchlässiges Gehäuse für eine Lichtquelle aufweist und wobei ein äußerer röhrenförmiger Bereich eine Reaktionskammer aufweist.

16. Verfahren nach Anspruch 15, wobei die Reaktionskammer durch einen Abstand von 2 mm bis 15 cm zwischen der inneren Röhre und der äußeren Röhre definiert ist.

17. Verfahren nach einem der vorhergehenden Ansprüche, wobei der Triplett-Sensitizer mit einem molaren Verhältnis von 0,08 bis 0,35 Mol Triplett-Sensitizer/Mol Vitamin-D-Derivat vorliegt und wobei die Vitamin-D-Derivate in dem Lösungsmittel mit einer Konzentration im Bereich von 0,025 g - 0,1 g/ml Lösungsmittel gelöst sind.

18. Verfahren zur Herstellung von Calcipotriol oder Calcipotriolmonohydrat, umfassend in einem oder mehreren Schritten das Verfahren nach einem der vorhergehenden Ansprüche.

19. Verfahren zum Isomerisieren einer Lösung eines Vitamin-D-Derivats der allgemeinen Struktur IIaaa; 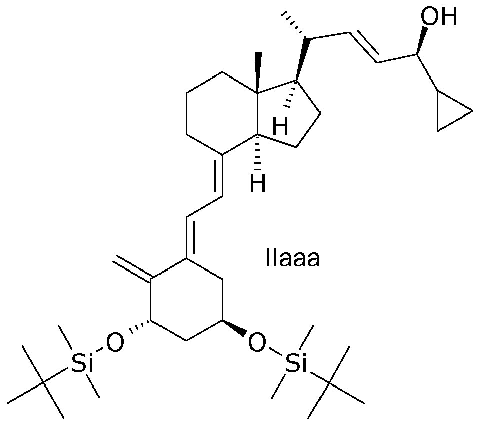 um ein Vitamin-D-Derivat der allgemeinen Struktur IIIaaa zu erhalten, 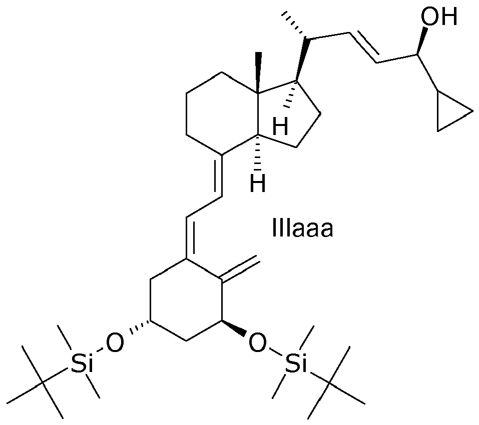 wobei das Verfahren das Bestrahlen einer Lösung eines Vitamin-D-Derivates der allgemeinen Struktur IIaa mit einer geeigneten Lichtquelle in Gegenwart eines Triplett-Sensitizers mit einer Triplett-Energie im Bereich von 150-270 kJ/mol umfasst;
wobei sich die Lösung in mehreren Durchläufen mit kontinuierlicher Strömung relativ zu der Lichtquelle in einem Durchfluss-Photoreaktor oder Photoreaktor mit kontinuierlicher Strömung bewegt;
**gekennzeichnet dadurch, dass** eine Fraktion der gesamten Lösung kontinuierlich und wiederholt von einem Reservoir durch den Durchfluss-Photoreaktor oder Photoreaktor mit kontinuierlicher Strömung zurück zu dem Reservoir zirkuliert.

20. Verfahren zur Herstellung einer pharmazeutischen Formulierung oder eines Medikaments, das Calcipotriol oder Calcipotriolmonohydrat enthält, wie eine Creme, eine Salbe oder ein Gel, umfassend das Verfahren nach einem der Ansprüche 1-19.

21. Verwendung eines Durchfluss-Photoreaktors oder Photoreaktors mit kontinuierlicher Strömung bei der Herstellung von Calcipotriol oder Calcipotriolhydrat.

22. Verwendung eines Durchfluss-Photoreaktors oder Photoreaktors mit kontinuierlicher Strömung bei der Herstellung von alpha-Calcidol oder Calcitriol.

23. Verwendung nach Anspruch 21, wobei der Durchfluss-Photoreaktor oder Photoreaktor mit kontinuierlicher Strömung ein im Wesentlichen axial-symmetrischer Durchfluss-Rohrreaktor ist, in dem ein längsgestrecktes Lampengehäuse bevorzugt zentral in einem inneren Bereich der Reaktorkammer plaziert ist und worin das Lampengehäuse longitudinal zur Fließrichtung angeordnet ist.

## Revendications

1. Procédé d'isomérisation d'une solution d'un dérivé de la vitamine D de structure générale IIa, IIb, IIc, IId ou IIe respectivement ; 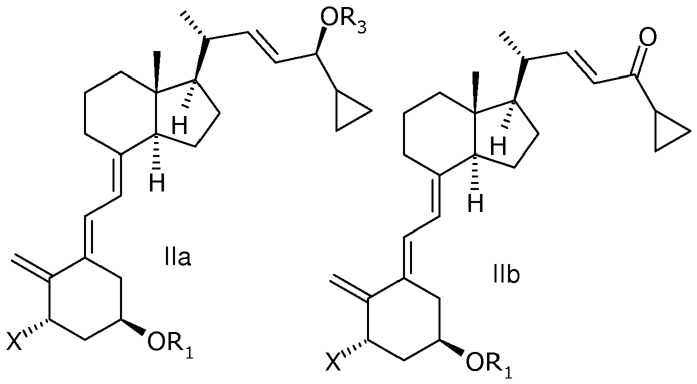 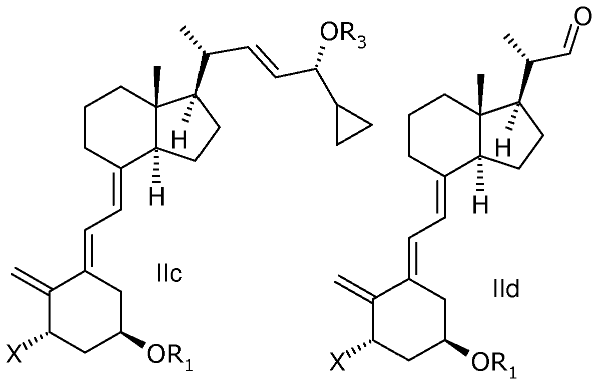 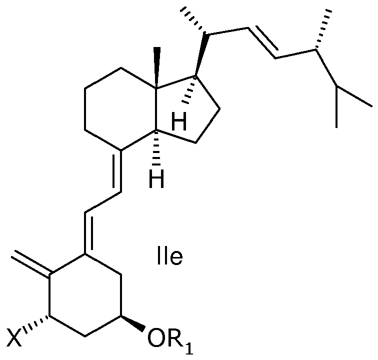 pour donner un dérivé de la vitamine D de structure générale IIIa, IIIb, IIIc, IIId ou IIIe respectivement ; 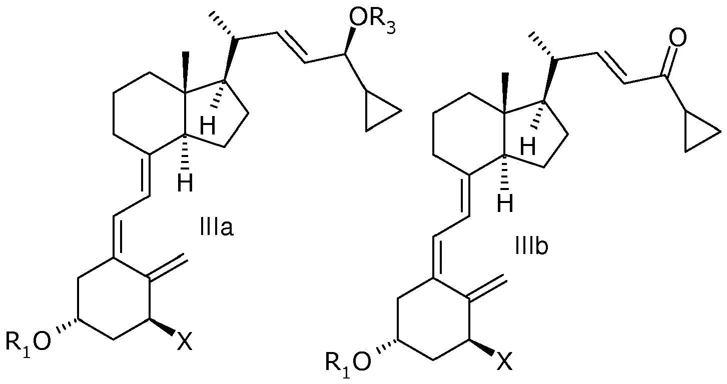 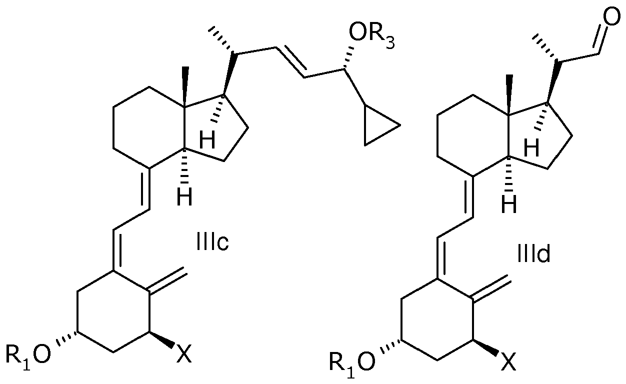 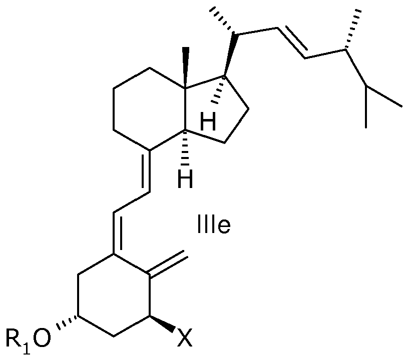 dans laquelle X représente un atome d'hydrogène ou -OR₂ ;
R₁, R₂ et R₃ peuvent être identiques ou différents et représentent indépendamment un atome d'hydrogène ou un groupe protégeant l'hydroxy ;
le procédé comprenant l'irradiation d'une solution d'un dérivé de la vitamine D de structure générale IIa, IIb, IIc, IId ou IIe respectivement,
avec une source de lumière appropriée, en présence d'un sensibilisateur à l'état triplet avec une énergie triplet dans la plage de 150 à 270 kJ/mol dans un photoréacteur à écoulement direct ou un photoréacteur à écoulement continu, dans lequel ladite solution circule dans un écoulement continu d'un passage unique ou de multiples passages par rapport à la source de lumière dans un photoréacteur à écoulement direct ou un photoréacteur à écoulement continu.

2. Procédé de production de calcipotriol {(5Z, 7E, 22E, 24S)-24-cyclopropyl-9,10-sécochola-5,7,10(19),22-tétraène-1α-3β-24-triol} ou de calcipotriol monohydraté comprenant les étapes de :
(i) isomérisation d'un dérivé de la vitamine D de structure générale IIaa 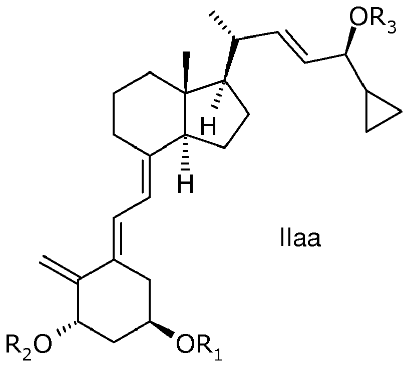 pour donner un dérivé de la vitamine D de structure générale IIIaa ; 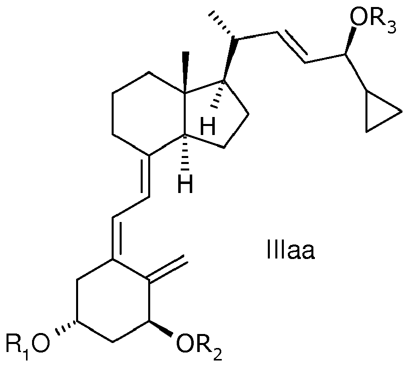 dans laquelle R₁, R₂ et R₃ peuvent être identiques ou différents et représentent indépendamment un atome d'hydrogène ou un groupe protégeant l'hydroxy ;
avec une source de lumière appropriée, en présence d'un sensibilisateur à l'état triplet avec une énergie triplet dans la plage de 150 à 270 kJ/mol ;
**caractérisé en ce que** ladite solution circule dans un écoulement continu d'un passage unique ou de multiples passages par rapport à la source de lumière dans un photoréacteur à écoulement direct ou un photoréacteur à écoulement continu ;
(ii) lorsque R₁ et/ou R₂ et/ou R₃ ne représentent pas un atome d'hydrogène, élimination du ou des groups protégeant l'hydroxy R₁ et/ou R₂ et/ou R₃ du composé de structure générale IIIa pour générer le calcipotriol ; et
(iv) cristallisation facultative du calcipotriol à partir d'un mélange d'un solvant organique et d'eau pour donner le calcipotriol monohydraté.

3. Procédé selon la revendication 1, dans lequel R₃ représente un atome d'hydrogène et X représente -OR₂.

4. Procédé selon l'une quelconque des revendications précédentes, dans lequel R₁ et R₂ représentent un groupe alkylsilyle ou un atome d'hydrogène.

5. Procédé selon l'une quelconque des revendications précédentes, dans lequel R₁ et R₂ représentent un groupe *tert*-butyldiméthylsilyle et R₃ représente un atome d'hydrogène.

6. Procédé selon l'une quelconque des revendications précédentes, dans lequel le photoréacteur à écoulement direct ou le photoréacteur à écoulement continu est un réacteur à écoulement tubulaire essentiellement axi-symétrique dans lequel la solution circule parallèlement à l'axe longitudinal central.

7. Procédé selon l'une quelconque des revendications précédentes, dans lequel la solution est collectée et mise en recirculation de multiples fois dans le photoréacteur à écoulement direct ou le photoréacteur à écoulement continu.

8. Procédé selon l'une quelconque des revendications précédentes, dans lequel une fraction de la solution totale est mise en circulation de manière continue et répétée à partir d'un ou plusieurs réservoirs dans le photoréacteur à écoulement direct ou le photoréacteur à écoulement continu et de nouveau dans le ou les réservoirs, dans lequel ou lesquels la solution est facultativement mélangée et la température est contrôlée dans ledit ou lesdits réservoirs.

9. Procédé selon l'une quelconque des revendications précédentes, dans lequel la source de lumière comprend une lampe à mercure moyenne pression dopée au fer.

10. Procédé selon l'une quelconque des revendications précédentes, dans lequel la source de lumière fournit une lumière UV, en particulier dans la plage de 300 à 340 nm.

11. Procédé selon la revendication 9 ou 10, dans lequel la lampe à mercure moyenne pression fonctionne avec un courant électrique de 3 à 7 kW.

12. Procédé selon l'une quelconque des revendications précédentes, dans lequel le sensibilisateur à l'état triplet est choisi dans le groupe constitué par l'anthracène, le 9-acétylanthracène,l'acide anthracène-9-carboxylique, l'anthracène-carboxaldéhyde, la phénazine, l'acide anthracène-9-sulfonique, la 4,4-bis(diméthoxy)thiobenzophénone, la 4,4-bis(diméthylamino)benzophénone, la 4,4-bis(diméthylamino)thiobenzophénone, la 4,4-bis(diméthoxy)benzophénone, et le 9,10-diphénylanthracène, ou des mélanges de ceux-ci.

13. Procédé selon l'une quelconque des revendications précédentes, dans lequel le solvant est choisi dans le groupe constitué par le dichlorométhane, l'éther de tert-butylméthyle, le tétrahydrofurane, le dioxane, le diméthoxyéthane, l'hexane, l'heptane, le toluène, la triéthylamine, ou des mélanges de ceux-ci.

14. Procédé selon l'une quelconque des revendications précédentes, dans lequel l'isomérisation est réalisée à une température de 0 à 35 °C sous une atmosphère inerte.

15. Procédé selon l'une quelconque des revendications 6 à 14, dans lequel le réacteur à écoulement tubulaire essentiellement axi-symétrique comprend au moins deux espaces tubulaires concentriques coaxialement alignés, tels que des cylindres ou des tubes se prolongeant de manière longitudinale situés l'un dans l'autre, dans lequel un espace tubulaire interne fournit un logement perméable à la lumière pour une source de lumière, et dans lequel un espace tubulaire externe fournit une chambre réactionnelle .

16. Procédé selon la revendication 15, dans lequel la chambre réactionnelle est définie par un espacement entre le tube de délimitation interne et le tube de délimitation externe de 2 mm à 15 cm.

17. Procédé selon l'une quelconque des revendications précédentes, dans lequel le sensibilisateur à l'état triplet est présent à un rapport molaire de 0,08 à 0,35 moles de sensibilisateur à l'état triplet/mole de dérivé de la vitamine D, et dans lequel les dérivés de la vitamine D sont dissous dans le solvant à une concentration comprise dans la plage allant de 0,025 g à 0,1 g/ml de solvant.

18. Procédé de préparation de calcipotriol ou de calcipotriol monohydraté, en une ou plusieurs étapes, le procédé selon l'une quelconque des revendications précédentes.

19. Procédé d'isomérisation d'une solution d'un dérivé de la vitamine D de structure générale IIaaa ; 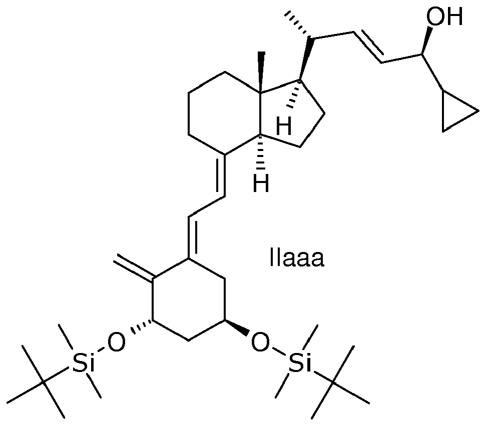 pour donner un dérivé de la vitamine D de structure générale IIIaaa, 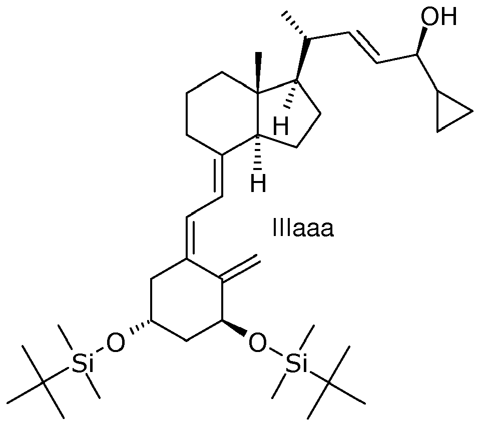 le procédé comprenant l'irradiation d'une solution d'un dérivé de la vitamine D de structure générale IIaaa, avec une source de lumière appropriée, en présence d'un sensibilisateur à l'état triplet avec une énergie triplet dans la plage de 150 à 270 kJ/mol ;
dans lequel ladite solution circule dans un écoulement continu de multiples passages par rapport à la source de lumière dans un photoréacteur à écoulement direct ou un photoréacteur à écoulement continu ;
**caractérisé en ce qu'**une fraction de la solution totale est mise en circulation de manière continue et répétée à partir d'un réservoir dans le photoréacteur à écoulement direct ou le photoréacteur à écoulement continu et de nouveau dans le réservoir.

20. Procédé de préparation d'une formulation pharmaceutique ou d'un médicament contenant du calcipotriol ou du calcipotriol monohydraté, telle qu'une crème, une pommade ou un gel comprenant le procédé selon l'une quelconque des revendications 1 à 19.

21. Utilisation d'un photoréacteur à écoulement direct ou un photoréacteur à écoulement continu pour la préparation de calcipotriol ou de calcipotriol hydraté.

22. Utilisation d'un photoréacteur à écoulement direct ou un photoréacteur à écoulement continu pour la préparation d'alpha-calcidol ou de calcitriol.

23. Utilisation selon la revendication 21 dans lequel le photoréacteur à écoulement direct ou le photoréacteur à écoulement continu est un réacteur à écoulement tubulaire essentiellement axi-symétrique dans lequel un logement de lampe allongé est placé essentiellement de manière centrale dans une partie interne de la chambre réactionnelle et dans lequel ledit logement de lampe est aligné de manière longitudinale par rapport au sens de l'écoulement.
